# EUROPEAN PATENT APPLICATION

(11) **EP 3 040 325 A1**
(43) Date of publication of application: **06.07.2016**
(21) Application number: 14200714.5
(22) Date of filing: 31.12.2014
(51) Int. Cl.: C07C 1/20, C07C 2/86, C07C 7/148, C10G 9/36, C07C 11/02

(54) **Process for preparing ethylene, propylene and isoprene**

(71) Applicant: Shell Internationale Research Maatschappij B.V., 2596 HR Den Haag (NL)
(72) Inventor: VAN WESTRENEN, Jeroen, 1031HW Amsterdam (NL); MARSMAN, Jeroen Gerard, 1031HW Amsterdam (NL); CHEWTER, Leslie Andrew, 1031HW Amsterdam (NL)
(74) Representative: Matthezing, Robert Maarten

(57) **Abstract**

A process for preparing ethylene, propylene and isoprene, comprising converting oxygenates to olefins, cracking a hydrocarbon stream and converting the isobutenes formed in these or additional steps to isoprene.

## Description

### Field of the invention

The invention relates to a process for preparing ethylene, propylene and isoprene.

### Background to the invention

Isoprene is a valuable compound that can be used for the production of polyisoprene, a synthetic version of natural rubber. Industrially, isoprene can be obtained by extracting it from the C5 fraction of pyrolysis gasoline, a byproduct of the steam cracking of naphtha. The yield of isoprene from a steam cracking process is, however, very low, typically in the range of from 1 to 3 % of the ethylene produced from the process. Moreover, the isolation of isoprene from pyrolysis gasoline is complicated, since it requires removal of other compounds such as cyclopentadiene, piperylenes and 1,3-pentadiene.

As an alternative, the condensation of formaldehyde and isobutene (Prins reaction) has been proposed for the production of isoprene. In this reaction, formaldehyde and isobutene are condensed in an aqueous phase comprising an acid catalyst to form intermediate products like 4,4-dimethyl-m-dioxane and 3-methyl-1,3-butanediol. These compounds can then be decomposed into isoprene. In this process, carbon monoxide, carbon dioxide, formaldehyde, acetaldehyde and n-butenes are formed as byproducts.

In WO 2009/065898 a process is proposed wherein the production of ethylene, propylene, and isoprene from butane is combined. Butanes are fed to a de-isobutaniser to be separated into n-butanes and isobutane. The n-butanes are fed to a steam cracker, together with ethane and propane, to form ethylene and propylene. Isobutane from the de-isobutaniser is dehydrogenated to form isobutene and then condensed with formaldehyde to form isoprene.

A disadvantage of the process as described in WO 2009/065898 is that the byproducts formed in the process, in particular n-butenes, are not converted into additional ethylene and propylene.

### Summary of the invention

It has now been found that the production of lower olefins such as ethylene and propylene, can advantageously be combined with the production of isoprene. In the process according to the invention, isobutene produced as by-product in the conversion of oxygenate into lower olefins is used along with isobutene produced in a steam cracking unit for the manufacture of isoprene by condensing the isobutene with formaldehyde.

Accordingly, the present invention relates to a process for preparing ethylene, propylene and isoprene, the process comprising the following steps: a) contacting the oxygenate with a molecular sieve-comprising catalyst, at a temperature in the range of from 350 to 1000 °C to produce an oxygenate conversion effluent comprising ethylene, propylene and C4+ hydrocarbons including C4+ paraffins and C4+ olefins including isobutene; b) cracking a hydrocarbon feedstock under steam cracking conditions to produce a cracked effluent comprising ethylene, propylene and C4+ hydrocarbons including C4+ olefins including isobutene; c) combining the oxygenate conversion effluent and the cracked effluent to form a combined effluent; d) subjecting the combined effluent to one or more separation steps such that at least an olefin product stream comprising ethylene and/or propylene, a stream comprising C5+ hydrocarbons and a stream comprising C4 hydrocarbons including butane, n-butenes and isobutene, are obtained; e) removing butadiene from the stream comprising C4 hydrocarbons by extraction or selective hydrogenation to form a first raffinate stream comprising the remaining C4 hydrocarbons; f) selectively removing at least part of the isobutene from the first raffinate stream by supplying an alcohol and at least part of the first raffinate stream to an etherification reaction zone comprising an etherification catalyst and reacting at least part of the isobutene with the alcohol to obtain an etherification product stream comprising an alkyl tert-butyl ether and separating at least part of the etherification product stream into an alkyl tert-butyl ether-enriched stream and an isobutene-depleted second raffinate stream; g) decomposing at least part of the alkyl tert-butyl ether into isobutene and the corresponding alcohol; and h) reacting at least part of the isobutene with formaldehyde to produce isoprene.

An important advantage of the process according to the invention is that by-products formed in the reaction of formaldehyde with isobutene, in particular normal butenes, can advantageously be recycled to the oxygenate conversion step a) to increase the yield of lower olefins.

A further advantage of the process according to the invention is that the formaldehyde used for condensation with isobutene may be prepared from methanol, i.e. the same oxygenate as is preferably used as feedstock for oxygenate conversion step a). Moreover, the preparation of formaldehyde from methanol may be carried out in such a way that there is a net production of hydrogen. This can be done by carrying out this step with a substoichiometric amount of oxygen and in the presence of a catalyst with a dehydrogenation functionality, for example a silver catalyst. The hydrogen thus produced may be advantageously used for partial hydrogenation of acetylene or dienes formed as byproduct in oxygenate conversion step a).

### Brief description of the drawings

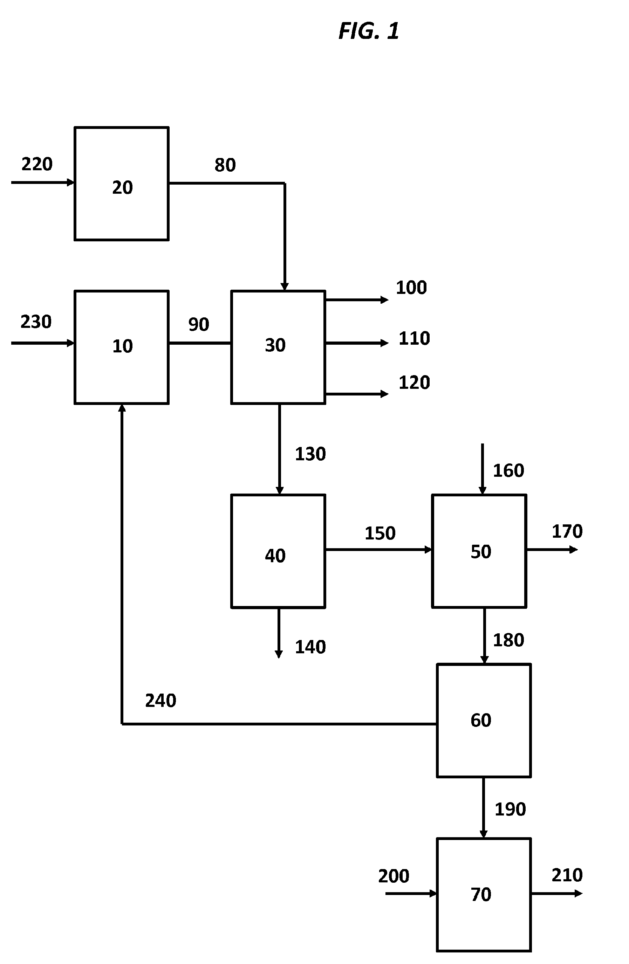

Figure 1 schematically shows a process according to the invention.

### Detailed description of the invention

In the process according to the invention, ethylene, propylene and isoprene are prepared, in a process comprising several steps. In a first step, an oxygenate is converted into lower olefins, i.e. ethylene and propylene, by contacting the oxygenate with a molecular sieve-comprising catalyst at a temperature in the range of from 350 to 1000 °C (oxygenate conversion step a)). In a second step, a hydrocarbon feedstock is converted into olefins and other cracked products. By combining the effluents, the separation section for these two processes can be combined. This invention provides a process for removing the valuable isobutene and converting it into even more valuable isoprene. The use of the combined processes allows the butenes and other streams to be recycled to produce additional olefins which results in improved yields.

Reference herein to an oxygenate is to a compound comprising at least one alkyl group that is covalently linked to an oxygen atom. Preferably, the at least one alkyl group has up to five carbon atoms, more preferably up to four, even more preferably one or two carbon atoms, most preferably is methyl. Mono-alcohols and dialkylethers are particularly suitable oxygenates. Methanol, dimethylether and mixtures thereof are examples of particularly preferred oxygenates. Most preferably, the oxygenate is methanol.

Oxygenate conversion step a) is carried out by contacting the oxygenate with a molecular sieve-comprising catalyst at a temperature in the range of from 350 to 1000 °C, preferably of from 350 to 750 °C, more preferably of from 450 to 700 °C, even more preferably of from 500 to 650 °C. The conversion may be carried out at any suitable pressure, preferably at a pressure in the range of from 1 bar to 50 bar (absolute), more preferably of from 1 bar to 15 bar (absolute). A pressure in the range of from 1.5 to 4.0 bar (absolute) is particularly preferred.

Any molecular sieve comprising catalyst known to be suitable for the conversion of oxygenates, in particular alkanols and dialkylethers, into lower olefins may be used. Preferably the catalyst comprises a molecular sieve having a 8-, 10- or 12-ring structure and an average pore size in the range of from 3 Å to 15 Å. Examples of suitable molecular sieves are silicoaluminophosphates (SAPOs), aluminophosphates (AlPO), metal-substituted aluminophosphates or metal-substituted silicoaluminophosphates. Preferred SAPOs include SAPO-5, -8, -11, -17, -18, -20, -31, -34, -35, -36, -37, -40, -41, -42, -44, -47 and -56. SAPO-17, -18, -34, -35, and -44 are particularly preferred.

A particular suitable class of molecular sieves are zeolites, more in particular a zeolite with a 10-membered ring structure. Zeolite-comprising catalysts are known for their ability to convert higher olefins into lower olefins, in particular to convert C4+ olefins into ethylene and/or propylene. Suitable zeolite-comprising catalysts include those containing a zeolite of the ZSM group, in particular of the MFI type, such as ZSM-5, the MTT type, such as ZSM-23, the TON type, such as ZSM-22, the MEL type, such as ZSM-11, or the FER type. Other suitable zeolites are for example zeolites of the STF-type, such as SSZ-35, the SFF type, such as SSZ-44 and the EU-2 type, such as ZSM-48. Preferably, the catalyst comprises at least one zeolite selected from MFI, MEL, TON and MTT type zeolites, more preferably at least one of ZSM-5, ZSM-11, ZSM-22 and ZSM-23 zeolites.

The zeolite in the oxygenate conversion catalyst is preferably predominantly in the hydrogen form. Preferably at least 50 wt%, more preferably at least 75 wt%, even more preferably at least 95 wt%, still more preferably at least 100 wt% of the zeolite is in the hydrogen form.

The molecular sieve-comprising catalyst may further comprise a binder material such as for example silica, alumina, silica-alumina, titania, or zirconia, a matrix material such as for example a clay, and/or a filler.

Besides lower olefins, C4+ hydrocarbons including C4+ paraffins and C4+ olefins are formed as by-product. Thus, an oxygenate conversion effluent comprising ethylene, propylene and C4+ hydrocarbons is produced in step a). Typically, C4+ paraffins such as isobutane, n-butane, n-pentane, iso-pentane, and C4+ olefins such as isobutene, n-butenes, n-pentenes, iso-pentenes and C5+ naphthenes such as cyclopentane and cyclopentene will be present in the oxygenate conversion effluent. Small amounts of dienes like butadienes may be present in the effluent.

In oxygenate conversion step a), not only lower olefins and C4+ hydrocarbons, but also water is formed. The effluent therefore also comprises water.

In step b) a hydrocarbon feed is cracked under steam cracking conditions to produce a cracked effluent. Steam cracking processes that can be applied in step (b) include any process known in the art. Suitable steam cracking processes to be used in step (b) are, for example, described in US 3,365,387 and US 4,061,562. In a steam cracking process the hydrocarbons are pyrolyzed in the presence of steam to form light olefins such as ethylene and propylene, and heavier products such as pyrolysis gas oil and pyrolysis gasoline.

In the steam cracking process in step (b), the hydrocarbon feedstock is contacted with steam at a temperature and pressure to form a cracked effluent which comprises olefins such as ethylene and propylene, including butadiene.

Suitably, the steam cracking process in step (b) is conducted at a temperature from 750 to 920 °C, preferably from 760 to 900 °C, more preferably from 770 to 890°C, even more preferably from 780 to 880°C; and a pressure of from 1 to 5 bara, preferably from 1.1 to 2.5 bara, more preferably from 1.5 to 2.5 bara.

The hydrocarbon feedstock to be steam cracked can be chosen from a variety of petroleum fractions, and it is preferably paraffinic in nature. Preferably, the hydrocarbon feedstock comprises at least one of ethane, propane, butane and/or at least one of a naphtha, kerosene, gasoil or hydrowax.

In step (c), at least part of the oxygenate conversion effluent as obtained in step (a) and at least part of the cracked effluent which comprises olefins as obtained in step (b) are combined to form a combined effluent stream comprising at least ethylene, propylene and a C4 hydrocarbon fraction comprising butadiene.

In step d), the combined effluent is subjected to one or more separation steps such that at least one or more olefin product streams comprising ethylene and/or propylene and a stream comprising C4 hydrocarbons is obtained. Water may be separated from the combined effluent by means known in the art, for example by cooling the effluent by indirect heat exchanged followed by a water quench tower. After water separation, an olefinic stream is obtained that may be separated into different fractions by means known in the art. In one embodiment, a fraction comprising mainly ethylene is first separated from such olefinic stream in a de-ethaniser and a fraction mainly comprising propylene is separated from the bottoms of the de-ethaniser in a de-propaniser. The bottoms of the de-propaniser contain the C4+ hydrocarbons. Alternatively, a fraction comprising both ethylene and propylene may be separated from the olefinic stream to obtain an olefinic product stream comprising both ethylene and propylene.

The stream comprising C4 hydrocarbons that is obtained in step d) may be the C4+ hydrocarbon fraction obtained as bottoms of a de-propaniser. Preferably, the stream comprising C4 hydrocarbon obtained in step d) does not comprise substantial amounts of C5+ hydrocarbons. Therefore, C5+ hydrocarbons are preferably separated from the C4+ hydrocarbon fraction as obtained in a de-propaniser, typically in a de-butaniser.

At least part of compounds other than isobutene are first removed from the stream comprising C4 hydrocarbons or at least part of the isobutene is first selectively removed from the stream comprising C4 hydrocarbons and then reacted with formaldehyde in step h). More preferably, at least part of the isobutene is selectively removed from the stream comprising C4 hydrocarbons obtained in step e) to obtain an isobutene-depleted C4 hydrocarbon stream. The selectively removed isobutene is then reacted with formaldehyde in step h)**.**

In an embodiment of the invention, at least part of the isobutene is selectively removed from the stream comprising C4 hydrocarbons by supplying an alcohol and at least part of the stream comprising C4 hydrocarbons to an etherification reaction zone comprising an etherification catalyst and reacting, in the etherification reaction zone, at least part of the isobutene with the alcohol to obtain an etherification product stream comprising an alkyl tertiary-butyl ether. The etherification product stream is then separated into an alkyl tertiary-butyl ether-enriched stream and the isobutene-depleted C4 hydrocarbon stream.

The alcohol is preferably an alcohol selected from the group consisting of methanol, ethanol and a mixture thereof. More preferably, the alcohol is methanol and an etherification product stream comprising methyl tertiary-butyl ether is obtained.

The stream comprising C4 hydrocarbons is partially hydrogenated in order to reduce the amount of any butadienes or other dienes before being supplied to the etherification reaction zone. In another embodiment, the butadiene is extracted from the C4 hydrocarbons stream and recovered as a product.

Etherification of isobutene to form an alkyl tertiary-butyl ether is well-known in the art. Any catalyst and process conditions known to be suitable for such etherification may be used. Typically, the etherification catalyst is an acid catalyst. Preferably, the etherification catalyst is a protonated cation-exchange resin or a heteropolyacid promoted by a metal. A particularly preferred catalyst is Amberlyst-15.

Preferably, the etherification reaction is carried out at a temperature in the range of from 40 to 100 °C, more preferably of from 50 to 85 °C. The reaction may be carried out at any suitable pressure, preferably in the range of from 1 to 20 bar (absolute), more preferably of from 5 to 15 bar (absolute).

At least part of the etherification product stream is separated into an alkyl tertiary-butyl ether-enriched stream and an isobutene-depleted C4+ hydrocarbon stream. This may be done by any suitable means known in the art, for example by distillation.

The alkyl tertiary-butyl ether in the alkyl tertiary-butyl ether-enriched stream may be converted into the alcohol and isobutene by means of catalytic cracking prior to reacting the isobutene with formaldehyde. The cracking of a tertiary alkyl ether into its corresponding alcohol and tertiary iso-olefin is well-known in the art. The cracking may be carried out in any suitable way known in the art. In the cracking step, preferably an acid catalyst is used. Preferred cracking catalysts include acid cation-exchange resins, heteropolyacids, metal oxides such as for example alumina or silica-alumina. The catalytic cracking is preferably carried out at a temperature in the range of from 100 to 250 °C, more preferably of from 120 to 200 °C. The pressure is preferably in the range of from 1 to 10 bar (absolute).

The alcohol obtained in catalytic cracking of the alkyl tertiary butyl ether may be recycled to the etherification reaction zone. In case the alcohol is methanol and methanol is used as the oxygenate feed in step a), the methanol may be recycled to oxygenate conversion step a).

In step h) of the process according to the invention, at least part of the isobutene produced in oxygenate conversion step a) and in cracking step b) is reacted with formaldehyde to produce isoprene.

Processes for the manufacture of isoprene by reacting isobutene with formaldehyde by means of condensation followed by decomposition of condensation products formed to isoprene are well-known in the art. Step h) may comprise any suitable reaction conditions known in the art. Suitable reaction conditions are for example disclosed in WO 2009/065898, GB 1 370 899, US 3,972,955 and EP 1 614 671. Typically, isobutene and formaldehyde are converted into isoprene by reacting isobutene with formaldehyde in an aqueous phase in the presence of an acid catalyst to give intermediate products like 4,4-dimethyl-m-dioxane and 3-methyl-1,3-butanediol. The intermediate products are decomposed in a gas phase to isoprene. The reaction of isobutene with formaldehyde to form 4,4-dimethyl-m-dioxane and 3-methyl-1,3-butanediol is known as the Prins reaction.

In step h), n-butenes are typically produced as byproduct. Isoprene is usually removed from the aqueous phase by means of distillation. Thus, a product stream comprising isoprene and a stream comprising n-butenes and acetaldehyde is recovered. It is preferred to recycle at least part of the stream comprising normal butenes to oxygenate conversion step a) in order to increase the yield of lower olefins.

A homogeneous or heterogeneous acid catalyst may be used in step h). Any suitable catalyst known in the art may be used. In case a homogeneous catalyst is used, i.e. a liquid acid, the acid preferably has a boiling point above the boiling point of isoprene so that it will remain in the aqueous phase during distillation of isoprene from that phase.

The process according to the invention preferably further comprises the following step:
i) conversion of methanol into formaldehyde by means of catalytic partial oxidation and/or catalytic dehydrogenation and using the formaldehyde thus formed in step h) for the conversion of isobutene into isoprene.

The methanol may be external methanol. Preferably, the methanol is methanol at least partly obtained in catalytic cracking of methyl alkyl butyl ether formed in the etherification reaction zone.

The conversion of methanol into formaldehyde by means of catalytic partial oxidation and/or catalytic dehydrogenation is well-known in the art.

Any suitable catalyst and reaction conditions known in the art may be used is step i). Preferably, the catalyst is a silver catalyst or a metal oxide catalyst. More preferably, step i) is carried out such that hydrogen is produced in step i) by using a catalyst having dehydrogenation and oxidation functionality, preferably a silver catalyst. Step i) then comprises catalytic dehydrogenation and catalytic partial oxidation of methanol by contacting methanol in the presence of steam and a molecular oxygen-containing gas with a catalyst having dehydrogenation and oxidation functionality. In order to achieve partial oxidation, a substoichiometric amount of oxygen is used.

Preferably, hydrogen produced in step i) is used to partially hydrogenate dienes or acetylene produced in oxygenate conversion step a).

Isobutene obtained after catalytic cracking of the alkyl tertiary-butyl ether may be reacted with formaldehyde by feeding formaldehyde and isobutene that has been separated from the alcohol obtained in catalytic cracking to a reaction zone suitable for condensation of isobutene with formaldehyde. In case the alcohol is methanol and methyl tertiary-butyl ether is formed in the etherification reaction zone, it is preferred to combine the catalytic cracking of methyl tertiary-butyl ether with the conversion of methanol obtained in the cracking into formaldehyde. Such combining may for example be done by feeding both methyl tertiary-butyl ether and a source of oxygen to a reaction zone comprising both a catalytic cracking function and a catalytic oxidation function. Reaction conditions and catalysts suitable for such combined catalytic cracking and partial oxidation are known in the art and for example disclosed in US 5,177,290. Any suitable reaction conditions and catalysts known in the art may be used. In a subsequent process step, the isobutene and formaldehyde formed are reacted to produce isoprene. Such process step is known in the art and for example disclosed in US 5,177,290.

Preferably, at least part of the isobutene-depleted C4 hydrocarbon stream obtained after selective removal of isobutene from the stream comprising C4 hydrocarbons is recycled to step a). In case of such recycle, preferably part of the isobutene-depleted C4 hydrocarbon stream is purged from the process in order to avoid too much accumulation of paraffins in the recycle stream. Instead of recycling part of the isobutene-depleted C4 hydrocarbon stream to step a), it may be advantageous to subject this stream to an olefin cracking step wherein C4+ olefins in this stream are cracked to ethylene and propylene. Olefin cracking is known in the art and is often applied to the C4 effluent of an oxygenate-to-olefins process wherein a molecular sieve-comprising catalyst is used that does not or hardly catalyse the conversion of C4+ olefins to ethylene and propylene, such as for example SAPO-comprising catalysts. In an embodiment of the invention, a SAPO-containing catalyst, more preferably a SAPO-34-containing catalyst, is used in step a) and at least part of the isobutene-depleted C4 hydrocarbon stream is subjected to an olefin cracking step. Suitable catalysts and process conditions for olefin cracking are known in the art.

Preferably, the process further comprises conversion of the isoprene obtained in step h) into polyisoprene and recovery of a polyisoprene product stream.

### Detailed description of the drawings

In Figure 1, an embodiment of the invention is schematically shown. Methanol or another oxygenate 230 is fed to oxygenate conversion reaction zone 10. The oxygenate is converted to olefins and other by-products which are fed via line 90 to separation section 30. A hydrocarbon feed 220 is fed to the steam cracking unit 20 where it is cracked to olefins and other pyrolysis products. The cracked products are passed via line 80 to the separation section 30. The combined effluent is separated into a number of streams, including an ethylene stream 100, a propylene stream 110, a C5+ stream 120 and a C4 stream 130. The C4 stream is fed to a butadiene removal section 40. The butadiene may be extracted or it may be selectively hydrogenated to convert it to mono-olefins. If it is extracted then the butadiene is removed via line 140. The first raffinate stream (C4 minus butadiene) is passed via line 150 to etherification reaction zone 50. An alcohol, for example, methanol or ethanol is fed via line 160. The first raffinate stream is contacted with the alcohol and produces an alkyl tert-butyl ether stream 180 and a second raffinate stream 170. The alkyl tert-butyl ether stream 180 is decomposed in decomposition unit 60 to form an alcohol stream 240 that can be recycled to the oxygenate conversion reaction zone 10 and an isobutylene stream 190 that can be fed to the isoprene production zone 70. Formaldehyde is fed via line 200 to the isoprene production zone and isoprene is produced via line 210.

In an alternative embodiment (not shown), cracking of MtBE into isobutene and methanol, partial oxidation of methanol to formaldehyde and condensation of isobutene and formaldehyde, may be combined in a single reaction zone.

## Claims

1. A process for preparing ethylene, propylene and isoprene, the process comprising the following steps:
a) contacting the oxygenate with a molecular sieve-comprising catalyst, at a temperature in the range of from 350 to 1000 °C to produce an oxygenate conversion effluent comprising ethylene, propylene and C4+ hydrocarbons including C4+ paraffins and C4+ olefins including isobutene;
b) cracking a hydrocarbon feedstock under steam cracking conditions to produce a cracked effluent comprising ethylene, propylene and C4+ hydrocarbons including C4+ olefins including isobutene;
c) combining the oxygenate conversion effluent and the cracked effluent to form a combined effluent;
d) subjecting the combined effluent to one or more separation steps such that at least an olefin product stream comprising ethylene and/or propylene, a stream comprising C5+ hydrocarbons and a stream comprising C4 hydrocarbons including butane, n-butenes and isobutene, are obtained;
e) removing butadiene from the stream comprising C4 hydrocarbons by extraction or selective hydrogenation to form a first raffinate stream comprising the remaining C4 hydrocarbons;
f) selectively removing at least part of the isobutene from the first raffinate stream by supplying an alcohol and at least part of the first raffinate stream to an etherification reaction zone comprising an etherification catalyst and reacting at least part of the isobutene with the alcohol to obtain an etherification product stream comprising an alkyl tert-butyl ether and separating at least part of the etherification product stream into an alkyl tert-butyl ether-enriched stream and an isobutene-depleted second raffinate stream;
g) decomposing at least part of the alkyl tert-butyl ether into isobutene and the corresponding alcohol; and
h) reacting at least part of the isobutene with formaldehyde to produce isoprene.

2. The process of claim 1, where the oxygenate is methanol.

3. The process of any of claims 1-2, wherein the molecular sieve-containing catalyst is a zeolite-comprising catalyst.

4. The process of claim 3, wherein the zeolite-comprising catalyst comprises at least one of ZSM-5, ZSM-11, ZSM-22 and ZSM-23 zeolites.

5. The process of any of claims 1-4, wherein the alcohol is methanol and the alkyl tert-butyl ether is methyl tert-butyl ether.

6. The process of any of claims 1-5, further comprising the following step:
i. conversion of methanol into formaldehyde by means of catalytic partial oxidation and/or catalytic dehydrogenation and using the formaldehyde thus formed in step h) for the conversion of isobutene into isoprene.

7. The process of claim 6, wherein step i) comprises catalytic dehydrogenation and catalytic partial oxidation of methanol by contacting methanol in the presence of steam and a molecular oxygen-containing gas with a catalyst having dehydrogenation and oxidation functionality and wherein hydrogen is produced in step i).

8. The process of claim 7, wherein the catalyst is a silver catalyst.

9. The process of claim 7 wherein the hydrogen is used to hydrogenate diolefins and/or acetylene present in the combined effluent stream.

10. The process of any of claims 6-9, wherein the methanol obtained in the decomposition is used for conversion into formaldehyde in step i).

11. The process of any of claims 6-9, wherein the alcohol obtained in the decomposition is recycled to the etherification reaction zone.

12. The process of any of claims 6-9, wherein the alcohol obtained in the decomposition is recycled to step a).

13. The process of any of claims 1-12, wherein normal butenes are formed in any of the steps as by-product, the process further comprising separating normal butenes thus-formed and recycling at least part of the separated normal butenes to step a).

14. The process of any of claims 1-13 further comprising recycling the second raffinate stream to step a) and/or to step b).

15. The process of any of claims 1-11, further comprising conversion of the isoprene obtained in step h) into polyisoprene and recovery of a polyisoprene product stream.

16. The process of any of claims 1-2, wherein the molecular sieve-containing catalyst is a SAPO-comprising catalyst.

17. The process of claim 16, wherein the SAPO-comprising catalyst comprises at least one of SAPO-17, -18, -34, - 35, and -44

18. The process of claims 16-17, wherein normal butenes are formed in any of the steps as by-product, the process further comprising separating normal butenes thus-formed and passing at least part of the separated normal butenes to an olefin cracking process.

19. The process of claims 16-17, where the isobutene-depleted second raffinate stream of step f) is recycled to step a) and/or to step b)
